# EUROPEAN PATENT APPLICATION

(11) **EP 1 531 001 A1**
(43) Date of publication of application: **18.05.2005**
(21) Application number: 04256802.2
(22) Date of filing: 04.11.2004
(51) Int. Cl.: B01J 37/00, B01J 23/648, B01J 23/652, B01J 27/057, C07C 57/00, C07C 255/00

(54) **Process for preparing mixed metal oxide catalyst**

(30) Priority: 17.11.2003 US 520758 P
(71) Applicant: Rohm and Haas Company, Philadelphia, Pennsylvania 19106-2399 (US); Thar Technologies, Inc., Pittsburgh, PA 15238 (US)
(72) Inventor: Gaffney, Anne Mae, West Chester, Pennsylvania 19380 (US); Martinez, Jose L., Gibsonia, Pennsylvania 15044 (US); Song, Ruozhi, Wilmington, Delaware 19809 (US)
(74) Representative: Buckley, Guy Julian

(57) **Abstract**

The present invention includes a process for preparing an improved catalyst having the steps of admixing compounds containing the components of the catalyst and at least one solvent to form a precursor; extracting the precursor with a supercritical stream to form a processed precursor, where the extracting step includes drying the precursor, atomizing the precursor, and combinations thereof; and calcining the processed precursor to form a catalyst. The process may include drying the precursor by introducing the precursor, which has been previously washed with an alcohol, such as ethanol or methanol, into a vessel and introducing the supercritical stream at a pressure and temperature above the critical point of the stream into the vessel. The process may include drying and atomizing the precursor by introducing the supercritical solvent into the vessel at a pressure and a temperature above critical point of the solvent and introducing the precursor into the extraction vessel through a nozzle. The process may also include drying and atomizing the precursor by introducing the precursor and the supercritical solvent into the vessel through a nozzle.

## Description

The present invention relates to an improved catalyst and a process for preparing an improved catalyst for the oxidation of alkanes or a mixture of alkanes and alkenes to their corresponding unsaturated carboxylic acids by vapor phase catalytic oxidation and to a process for the vapor phase catalytic oxidation of alkanes or a mixture of alkanes and alkenes to their corresponding unsaturated carboxylic acids. The present invention also relates to an improved catalyst a process for preparing an improved catalyst for producing unsaturated nitriles by subjecting alkanes or a mixture of alkanes and alkenes to vapor phase catalytic oxidation in the presence of ammonia.

### BACKGROUND OF THE INVENTION

Unsaturated carboxylic acids such as acrylic acid and methacrylic acid are industrially important as starting materials for various synthetic resins, coating materials and plasticizers. Commercially, the current process for acrylic acid manufacture involves a two-step catalytic oxidation reaction starting with a propene feed. In the first stage, propene is converted to acrolein over a modified bismuth molybdate catalyst. In the second stage, acrolein product from the first stage is converted to acrylic acid using a catalyst composed of mainly molybdenum and vanadium oxides. In most cases, the catalyst formulations are proprietary to the catalyst supplier, but the technology is well established. Moreover, there is an incentive to develop a single step process to prepare the unsaturated acid from its corresponding alkene. Therefore, the prior art describes cases where complex metal oxide catalysts are utilized for the preparation of unsaturated acid from a corresponding alkene in a single step.

Nitriles, such as acrylonitrile and methacrylonitrile, have been industrially produced as important intermediates for the preparation of fibers, synthetic resins, synthetic rubbers, and the like. The most popular method for producing such nitriles is to subject an olefin such as propene or isobutene to a catalytic reaction with ammonia and oxygen in the presence of a catalyst in a gaseous phase at a high temperature. Known catalysts for conducting this reaction include a Mo-Bi-P-O catalyst, a V-Sb-O catalyst, a Sb-U-V-Ni-O catalyst, a Sb-Sn-O catalyst, a V-Sb-W-P-O catalyst and a catalyst obtained by mechanically mixing a V-Sb-W-O oxide and a Bi-Ce-Mo-W-O oxide. However, in view of the price difference between propane and propene or between isobutane and isobutene, attention has been drawn to the development of a method for producing acrylonitrile or methacrylonitrile by an ammoxidation reaction wherein a lower alkane, such as propane or isobutane, is used as a starting material, and it is catalytically reacted with ammonia and oxygen in a gaseous phase in the presence of a catalyst.

The preparation of these catalysts relies on one of two methods broadly classed as precipitation and gel methods. In both methods, the components are admixed with each other in one or more solvents, the solvent is removed forming a precursor, and then the precursor is calcined to obtain the final catalyst. In the precipitation method, a secondary solvent, mechanism or device (e.g. a rotovap) is used to cause the catalyst to precipitate from the solvent (i.e., to remove the solvent and form the precursor). In the gel method, the gel is dried to remove the solvent(s) and form the precursor which is then calcined to obtain the final catalyst.

Most gels are extremely porous and have large surface areas and pore volumes which are desirable in a catalyst. Moreover, the drying step may significantly affect the quality of the catalyst. Drying a gel involves the removal of the interstitial liquid. When solvents are removed by simple evaporation, it is believed that the formation of a liquid-vapor interface within the gel results in surface tension in the pores of the gel and causes considerable shrinkage due to partial collapse of the gel network. The result is a hardened, porous mass that produces a poor catalyst that does not have high conversion rates of feed stock to the desired product, high selectivity for the desired products and the overall yield of the process in which the catalyst is used is inadequate.

One method used to overcome this problem of the liquid-vapor interface in drying gels has been the use of supercritical fluid extraction (SFE). When SFE is applied, the solvent in the wet gel is extracted by another solvent at conditions above the critical point of the mixture of two solvents. Despite the improvement over previous techniques, improvements in SFE still can be achieved thereby providing catalysts having larger surface areas, lower densities, larger pore size and larger pore volumes. While SFE has been applied to the synthesis of some types of catalysts, SFE has not previously been utilized to make mixed metal oxide catalysts. See, Handbook of Heterogeneous Catalysis, edited by Ertl, §2.1.4.2C (1997).

### STATEMENT OF THE INVENTION

The present invention relates to processes and systems for preparing an improved catalyst having the steps of admixing compounds containing the components of the catalyst and at least one solvent to form a precursor, extracting the precursor to form a processed precursor, and calcining the processed precursor to form a catalyst, wherein the extracting step comprises drying the precursor with a supercritical stream, atomizing the precursor, and combinations thereof.

The process may further comprise drying the precursor by introducing a precursor, which has been previously washed with an alcohol, such as ethanol or methanol, into a vessel and introducing a supercritical stream into the vessel at a pressure and temperature above the critical point of the supercritical stream.

The process may further comprise drying and atomizing the precursor by introducing the supercritical stream into the vessel and introducing the precursor into the vessel through a nozzle.

The process may further comprise drying and atomizing the precursor by introducing the precursor and the supercritical stream into the extraction vessel through a nozzle.

In another embodiment of the present invention, a catalyst for the oxidation of alkanes or a mixture of alkanes and alkenes to their corresponding unsaturated carboxylic acids by vapor phase catalytic oxidation, and prepared by the process of the present invention, may be a mixed metal oxide having an empirical formula: MoₐV_{b}E_{c}X_{d}Oₑ, wherein E is at least one element selected from the group consisting of Te and Sb, X is at least one element selected from the group consisting of Nb, Ta, Ti, Al, Zr, Cr, Mn, Fe, Ru, Co, Rh, Ni, Pd, Pt, Ag, B, Bi, In, Ce and W and mixtures thereof, and where a=1, b=0.01-1.0, c=0.01-1.0, d=0.01-1.0 and e is a number such that the total valency of the metal elements is satisfied.

Another embodiment of the present invention comprises a process for preparing an unsaturated carboxylic acid comprising subjecting an alkane, or a mixture of an alkane and an alkene, to vapor phase catalytic oxidation in the presence of a catalyst prepared by the aforesaid process for preparing an improved catalyst. Still another embodiment comprises a process for preparing an unsaturated nitrile comprising subjecting an alkane, or a mixture of an alkane and an alkene, to vapor phase catalytic oxidation in the presence of ammonia and a catalyst prepared by the aforesaid process for preparing an improved catalyst..

### BRIEF DESCRIPTION OF THE DRAWINGS

In the drawings:

Fig. 1 shows a drying and atomizing system suitable to carry out the process of the present invention.

Fig. 2 shows another embodiment of a drying and atomizing system suitable to carry out the process of the present invention.

Fig. 3 shows a chart comparing the production of acrylic acid with a rotovap dried catalyst and a catalyst dried using SFE in accordance with the present invention.

### DETAILED DESCRIPTION

The present invention relates to an improved process for preparing catalysts. More particularly, the process in accordance with the present invention comprises three general steps: precursor formation; extraction of the precursor solvent by drying, or drying and atomization, using a supercritical stream; and calcination. The process of the present invention is not limited to a particular type or composition of catalyst that is ultimately prepared and may be suitable for use in preparing all types of catalysts.

The process of the present invention is suitable for synthesizing various catalysts including, but not limited to, those disclosed in EP0630879 B1; JP07-053448; WO 00/09260; US5,380,933; WO 00/29106; JP2000-037623; US5,281,745; JP6-228073; and US6,043,185, which are each hereby incorporated herein by reference. For example, without limitation, the process of the present invention may be used to prepare a metal catalyst, such as a catalyst comprising a mixed metal oxide having the empirical formula AₐD_{b}E_{c}X_{d}Oₑ, wherein A is at least one element selected from the group consisting of Mo and W, D is at least one element selected from the group consisting of V and Ce, E is at least one element selected from the group consisting of Te, Sb and Se, and X is at least one element selected from the group consisting of Nb, Ta, W, Ti, Al, Zr, Cr, Mn, Fe, Ru, Co, Rh, Ni, Pt, Sb, Bi, B, In, Ce, As, Ge, Sn, Li, Na, K, Rb, Cs, Fr, Be, Mg, Ca, Sr, Ba, Ra, Hf, Ag, Pb, P, Pm, Eu, Gd, Dy, Ho, Er, Tm, Yb, Lu and mixtures thereof and a, b, c, d and e represent the atomic ratios of A, D, E, X, and O, respectively. Further, the stoichiometry of the catalyst is selected such that when a is 0.01 to 1.0, b is 0.003 to 1.0, c is 0.01 to 1.0, d is 0 to 1.0, and e is a number such that the total valency of the metal elements is satisfied. For example, the following stochiometry is possible: when a is 0.2 to 0.1, b is 0.1 to 0.5, c is 0.03 to 0.5, d is 0 to 0.9 and e is dependent on the oxidation states of said other elements. In addition, it is possible that, when a is 0.25 to 0.98, b is 0.15 to 0.45, c is 0.05 to 0.45, d is 0 to 0.85. In an alternative aspect, when a=1, b=0.01 to 1.0, c=0.01 to 1.0, d=0.01 to 1.0, and e is a number such that the total valency of the metal elements is satisfied.

The catalysts produced by this process may, for example, without limitation, have the following empirical formula: MoₐV_{b}E_{c}X_{d}Oₑ, wherein E is at least one element selected from the group consisting of Te and Sb, X is at least one element selected from the group consisting of Nb, Ta, Ti, Al, Zr, Cr, Mn, Fe, Ru, Co, Rh, Ni, Pd, Pt, Ag, B, Bi, In, Ce and W and mixtures thereof, and when a=1, b=0.01 to 1.0, c=0.01 to 1.0, d=0.01 to 1.0 and e is a number such that the total valency of the metal elements is satisfied.

The precursor formation step includes forming a slurry, solution or gel, by admixing metal compounds, preferably at least one of which contains oxygen, and at least one solvent in appropriate amounts to form the slurry, solution or gel. According to one method suitable for use in connection with the present invention, two solutions (Solution A and Solution B) are made, and the precursor is formed upon the admixing of the two solutions, where the precursor may be a gel, slurry, solution or colloidal suspension (all of which are generally referred to as "gels").

Suitable solvents include water; alcohols including, but not limited to, methanol, ethanol, propanol, and diols, etc.; as well as other polar solvents known in the art. Generally, water is often used. The water may be any water suitable for use in chemical syntheses including, without limitation, distilled water and de-ionized water. The amount of water present may be an amount sufficient to keep the components substantially in solution long enough to avoid or minimize compositional and/or phase segregation during the preparation steps. Accordingly, the amount of water will vary according to the amounts and solubilities of the materials combined, as would be readily determinable by persons of ordinary skill in the art. But, as stated above, the amount of water used is preferably sufficient to ensure that an aqueous solution (rather than a slurry) is formed at the time of mixing.

For example, when a mixed metal oxide of the formula MoₐV_{b}Te_{c}Nb_{d}Oₑ wherein the element A is Mo, the element M is V, the element N is Te and the element X is Nb, is to be prepared, an aqueous solution of niobium oxalate (e.g. "Solution A") may be added to an aqueous solution or slurry of ammonium heptamolybdate, ammonium metavanadate and telluric acid (e.g., "Solution B"), so that the atomic ratio of the respective metal elements would be in the prescribed proportions. Generally, upon the addition of the niobium solution to the other solution, a gel is formed.

The starting materials are not limited to those described above. A wide range of materials including, for example, oxides, nitrates, halides or oxyhalides, alkoxides, acetylacetonates, and organometallic compounds may be used. For example, ammonium heptamolybdate may be utilized for the source of molybdenum in the catalyst. However, compounds such as MoO₃, MoO₂, MoCl₅, MoOCl₄, Mo(OC₂H₅)₅, molybdenum acetylacetonate, phosphomolybdic acid and silicomolybdic acid may also be utilized instead of ammonium heptamolybdate. Similarly, ammonium metavanadate may be utilized for the source of vanadium in the catalyst. However, compounds such as V₂O₅, V₂O₃, VOCl₃, VCl₄, VO(OC₂H₅)₃, vanadium acetylacetonate and vanadyl acetylacetonate may also be utilized instead of ammonium metavanadate. The tellurium source may include telluric acid, TeCl₄, Te(OC₂H₅)₅, Te(OCH(CH₃)₂)₄ and TeO₂. The niobium source may include ammonium niobium oxalate, Nb₂O₅, NbCl₅, niobic acid or Nb(OC₂H₅)₅, as well as the more conventional niobium oxalate.

In the preparation of mixed metal oxide catalysts, the extraction step, in general, and the drying step in particular, may significantly affect the quality of the catalyst. The extraction step of the process according to the present invention involves supercritical fluid extraction and results in the production of an improved catalyst, including improvements to the conversion of feed stock to the desired product, the selectivity of the catalyst and the overall yield of the process.

Supercritical fluid extraction ("SFE") is a process which involves the use of a material in its supercritical state; namely that is above its critical temperature (Tc) and/or above its critical pressure (Pc). One skilled in the art would be generally familiar with the procedure of creating and using supercritical streams. Background information on SFE can be found in "Supercritical fluid methods and protocols" edited by Williams and Clifford (Humana Press, 2000) and "Supercritical fluid extraction: principles and practice" by McHugh (Knovel, 2001), both of which are hereby incorporated herein by reference.

Suitable supercritical streams include neat (i.e., substantially pure) materials and combinations of materials that are gases or liquids at ambient pressures or temperatures, wherein the supercritical stream is selected from the group consisting of carbon dioxide, water, ammonia, nitrogen, nitrous oxide, methane, ethane, ethylene, propane, butane, n-pentane, benzene, methanol, ethanol, isopropanol, isobutanol, monofluoromethane, trifluoromethane, chlorotrifluoromethane, monofluoromethane, hexafluoroethane, 1,1-difluoroethylene, 1,2-difluoroethylene,toluene, pyridine, cyclohexane, m-cresol, decalin, cyclohexanol, o-xylene, tetralin, aniline, acetylene, chlorotrifluorosilane, xenon, sulfur hexafluoride, propane and combinations thereof. Preferred supercritical streams include CO₂ lower alkanes, ethanol, methanol, other lower alcohols and combinations thereof. Most preferred supercritical streams include combinations of CO₂ with methanol or ethanol.

Without intending to be limited by theory, it is believed that the extraction step of the present process includes eliminating the internal tensile forces (e.g. surface tension) in the pores by preventing the formation of a liquid-vapor interface in the drying process while also forming fine particles of catalyst precursor. It is further believed that this elimination of the liquid-vapor interface prevents the break down of any structure in the precursor and is accomplished through solvent removal or drying with SFE and/or SFE combined with a process that also atomizes the precursor.

Generally, in SFE, the solvent in the precursor is extracted by a solvent (for example, without limitation, CO₂) or mixture of solvent and co-solvent (for example, without limitation, CO₂ + ethanol) above the critical temperature and critical pressure of the mixture. Neat supercritical solvents or supercritical solvents that are mixture of two or more cosolvents are collectively referred to as the supercritical stream. Under supercritical conditions there is no distinction between liquid and vapor and, therefore, it is believed that there is no surface tension or capillary pressure developed during drying. Without intending to be limited by theory, it is believed that the absence of surface tension allows the gel, in the preferred embodiment, to be dried with very little shrinkage and the gel matrix to remain nearly intact. Therefore, the resultant catalyst precursor formed by SFE is expected to have higher surface areas, lower densities, larger pore sizes and greater pore volumes.

The extraction step of the present invention includes SFE, or SFE with an atomization aspect. The catalyst precursor may be washed with an alcohol (such as, without limitation, methanol or ethanol) prior to conducting SFE. The alcohol wash step is not required, but helps to remove water from the precursor without detrimentally affecting the precursor. The precursor is introduced into a vessel, such as an extraction vessel, which is filled with alcohol (methanol or ethanol). This helps to minimize the evaporation of the solvent from the precursor during pressurization, which in turn helps prevent break down of any precursor structure. The selected supercritical stream, which may comprise, for example, carbon dioxide, would be pumped into the extraction vessel to the pressure above its critical pressure (Pc=73 bar in the case of CO₂).

In one embodiment of the process of the present invention, temperature of the system may be keep below the Tc to insure that the supercritical stream was a liquid at the beginning of the extraction step. For example, with CO₂ as the supercritical stream, the temperature of the system would be kept between 20-25°C, while the pressure would be above the critical point, as noted above. Under these conditions, CO₂ is in liquid state and CO₂ and lower alcohols are completely miscible. It is believed that, consequently, the alcohol in the gel would be replaced by the liquid CO₂. The temperature of the extraction vessel would be increased to a point above the Tc of the stream, 31°C in the case of CO₂, causing the transition from liquid CO₂ to supercritical CO₂. Finally, after a period of time, the pressure would be slowly reduced to ambient pressure at constant temperature.

In another embodiment of the process of the present invention, the supercritical stream may be preheated above its Tc before introduction into the extraction vessel. For example, CO₂ would be heated above 31°C before it was introduced into the extraction vessel. In this instance, preferably, the extraction would be carried out at conditions above the critical temperature and pressure for the combined CO₂ and alcohol. At these conditions the alcohol and CO₂ are completely miscible. Finally, after a period of time, the pressure would be slowly reduced to ambient pressure at temperatures above the critical temperature of the CO2.

Where the extraction step comprises both SFE drying combined with atomization, the precursor is made into very fine particles with high surface area, low density, large pore size and large pore volume of dried catalyst precursor that is ready for calcination. As used herein, the term "atomization" means the reduction or separation of liquids and/or solids to very small particles such that a spray or fine mist is formed therefrom. One benefit of atomization is that it is a one step process where the atomization and drying takes place in one step.

Atomization may be accomplished with any device or technique capable of producing fine particles of catalyst precursor in combination with SFE. For example, spray, ultrasonic, electrothermal, electrostatic, sonic, whistle, rotary, and flashing liquid atomizers, and combinations thereof, may be used to atomize the catalyst precursor. Preferably, however, a spray atomizer is used.

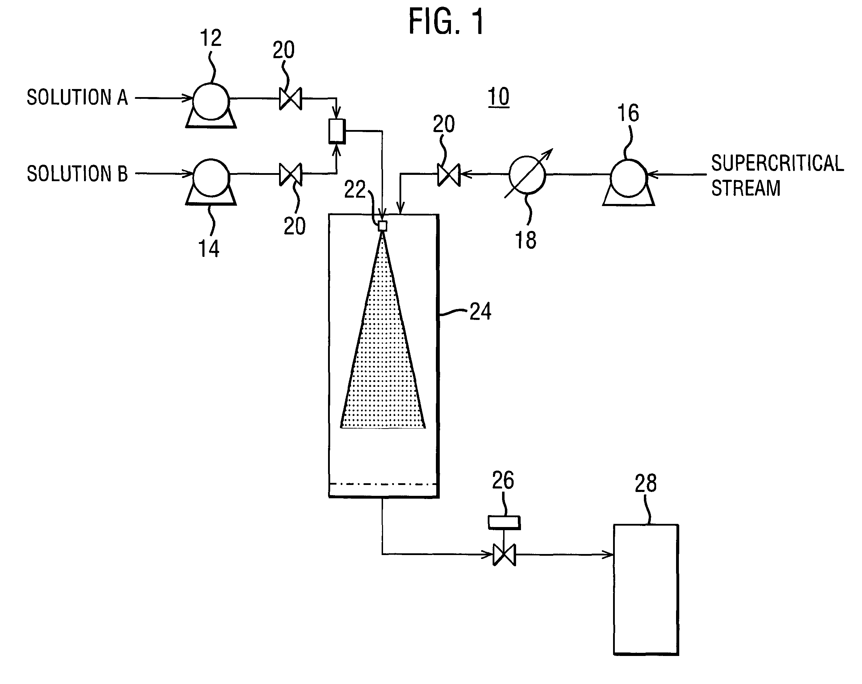

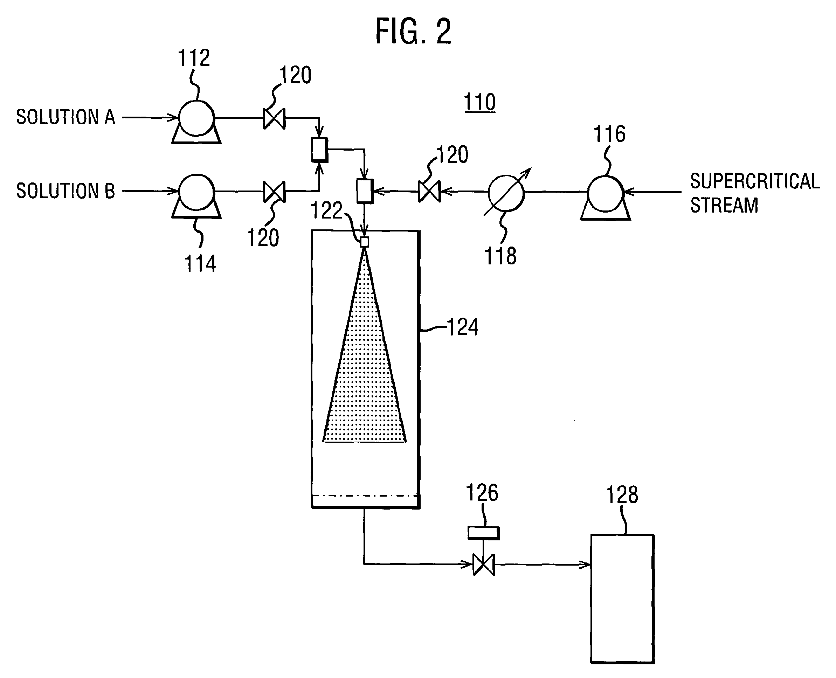

There are at least two techniques suitable for performing atomization in connection with the process of the present invention. In a first technique, shown in FIG. 1, the catalyst precursor to be atomized is introduced into a vessel containing the supercritical stream. In a second technique, shown in FIG. 2, the supercritical stream would also be used as a "spray enhancer", by combining the catalyst precursor and the supercritical stream before the combination is introduced (for example, sprayed), and thereby atomized, into a vessel. In both cases, the supercritical stream acts as anti-solvent, removing the solvent in the catalyst precursor, rapidly supersaturating the solution and precipitating the dispersed components of the precursor as fine particles. It is noted that the vessel may be any high pressure vessel, for example, but not limited to, an extraction vessel, that is capable of containing materials during high temperature and high pressure extraction processes, as described herein.

As seen in Fig. 1, the atomization system 10 includes pumps 12, 14 and 16, through which Solution A of the catalyst precursor, Solution B of the catalyst precursor, and the supercritical stream, respectively, are introduced. Solution A and B may be any suitable pair of solutions of catalytic precursors for making the desired catalyst, as previously described. The pumps 12, 14, 16 typically pressurize the components, including the supercritical stream to bring it above its critical pressure. Also, a heater 18 may be used, as needed, to raise the temperature of the supercritical stream. Numerous valves 20 may be used to control the flow of the various components. As shown in Fig. 1, Solutions A and B are admixed and sprayed, through a nozzle 22, into a suitable vessel, such as an extraction vessel 24, into which the supercritical stream has been previously introduced. In the extraction vessel 24, the supercritical stream extracts the precursor solvent while the nozzle 22 atomizes the precursor, resulting in fine particles of now dried catalyst precursor. The particles are collected and the supercritical stream and extracted solvents exit the extraction vessel 24 and are passed through a pressure reduction valve 26 to a separation vessel 28.

With reference to Fig. 2, it is noted that elements illustrated in Fig. 2, which correspond to the elements described above with respect to Fig. 1, have been designated by corresponding reference numerals increased by one hundred. The second embodiment of Fig. 2, as well as the various elements thereof, are constructed and designated for use in substantially the same manner as the embodiment of Fig. 1 and the elements thereof, unless otherwise stated.

The second technique for atomizing the catalyst precursor utilizes another embodiment of the atomization system 110, shown in FIG. 2, wherein the source of catalyst precursor and the source of the supercritical stream are connected to each other upstream from the point of introduction of the catalyst precursor into a vessel, such as an extraction vessel 124. Thus, the second technique involves combining the precursor with the supercritical stream before atomization at nozzle 122 and introduction into the extraction vessel 124. More particularly, the supercritical stream would also be used as a "spray enhancer" by combining the precursor formed from Solutions A and B and the supercritical stream before the combination is sprayed through the nozzle 122 into the vessel 124.

Parameters that may be adjusted to improve the particle size of the catalyst include, without limitation, flow rates of the precursor solutions, nozzle design and size, ratio of precursor to supercritical stream, flow rate of the supercritical stream/precursor, pressure and temperature in the vessel and in the area leading up to the nozzle.

After atomization and drying, the catalyst precursor may be calcined into any suitable form. The calcination may be conducted in an oxygen-containing atmosphere or in the substantial absence of oxygen, e.g., in an inert atmosphere or in vacuo. The inert atmosphere may be any material which is substantially inert, i.e., does not react or interact with, the catalyst precursor. Suitable examples include, without limitation, nitrogen, argon, xenon, helium, or mixtures thereof. Preferably, the inert atmosphere is argon or nitrogen. The inert atmosphere may flow over the surface of the catalyst precursor or may not flow thereover (i.e., a static environment). When the inert atmosphere does flow over the surface of the catalyst precursor, the flow rate can vary over a wide range, e.g., at a space velocity of from 1 to 500 hr⁻¹.

The calcination is usually performed at a temperature of from 350°C to 850°C, preferably from 400°C to 700°C, more preferably from 500°C to 640°C. The calcination is performed for an amount of time suitable to form the aforementioned catalyst. Typically, the calcination is performed for from 0.5 to 30 hours, preferably from 1 to 25 hours, more preferably for from 1 to 15 hours, to obtain the desired mixed metal oxide.

In a preferred mode of operation, the catalyst precursor is calcined in two stages. In the first stage, the catalyst precursor is calcined in an oxidizing environment (e.g. air) at a temperature of from 275°C to 400°C, such as from 275°C to 325°C for from 15 minutes to 8 hours, or even for from 1 to 3 hours. In the second stage, the material from the first stage is calcined in a non-oxidizing environment (e.g., an inert atmosphere) at a temperature of from 500°C to 700°C, such as for from 550°C to 650°C, for 15 minutes to 8 hours, or even for from 1 to 3 hours. Optionally, a reducing gas, such as, for example, ammonia or hydrogen, may be added during the second stage calcination.

In a particularly preferred mode of operation, the catalyst precursor in the first stage is placed in the desired oxidizing atmosphere at room temperature and then raised to the first stage calcination temperature and held there for the desired first stage calcination time. The heating ramp may be about 1 to about 10°C/min, for example about 2 to about 5°C/min. The atmosphere is then replaced with the desired non-oxidizing atmosphere for the second stage calcination; the temperature is raised to the desired second stage calcination temperature and held there for the desired second stage calcination time. The heating ramp may be about 1 to about 10°C/min, for example about 2 to about 5°C/min.

Although any type of heating mechanism, e.g., a furnace, may be utilized during the calcination, it is preferred to conduct the calcination under a flow of the designated gaseous environment. Therefore, it is advantageous to conduct the calcination in a bed with continuous flow of the desired gas(es) through the bed of solid catalyst precursor particles.

The present invention also provides a process for producing an unsaturated carboxylic acid, which comprises subjecting an alkane, or a mixture of an alkane and an alkene, to a vapor phase catalytic oxidation reaction in the presence of a catalyst made by this invention, to produce an unsaturated carboxylic acid.

In the production of such an unsaturated carboxylic acid, it is preferred to employ a starting material gas that contains steam. In such a case, as a starting material gas to be supplied to the reaction system, a gas mixture comprising a steam-containing alkane, or a steam-containing mixture of alkane and alkene, and an oxygen-containing gas, is usually used. However, the steam-containing alkane, or the steam-containing mixture of alkane and alkene, and the oxygen-containing gas may be alternately supplied to the reaction system. The steam to be employed may be present in the form of steam gas in the reaction system, and the manner of its introduction is not particularly limited.

Further, as a diluting gas, an inert gas such as nitrogen, argon or helium may be supplied. The molar ratio (alkane or mixture of alkane and alkene) : (oxygen) : (diluting gas) : (H₂O) in the starting material gas is preferably (1) : (0.1 to 10): (0 to 20) : (0.2 to 70), more preferably (1) : (1 to 5.0) : (0 to 10) : (5 to 40).

When steam is supplied together with the alkane, or the mixture of alkane and alkene, as starting material gas, the selectivity for an unsaturated carboxylic acid is distinctly improved, and the unsaturated carboxylic acid can be obtained from the alkane, or mixture of alkane and alkene, in good yield simply by contacting in one stage. However, the conventional technique utilizes a diluting gas such as nitrogen, argon or helium for the purpose of diluting the starting material. As such a diluting gas, to adjust the space velocity, the oxygen partial pressure and the steam partial pressure, an inert gas such as nitrogen, argon or helium may be used together with the steam.

As the starting material alkane it is preferred to employ a C₃₋₈ alkane, particularly propane, isobutane or n-butane; more preferably, propane or isobutane; most preferably, propane. According to the present invention, from such an alkane, an unsaturated carboxylic acid such as an α,β-unsaturated carboxylic acid can be obtained in good yield. For example, when propane or isobutane is used as the starting material alkane, acrylic acid or methacrylic acid will be obtained, respectively, in good yield.

In the present invention, as the starting material mixture of alkane and alkene, it is preferred to employ a mixture of C₃₋₈ alkane and C₃₋₈ alkene, particularly propane and propene, isobutane and isobutene or n-butane and n-butene. As the starting material mixture of alkane and alkene, propane and propene or isobutane and isobutene are more preferred. Most preferred is a mixture of propane and propene. According to the present invention, from such a mixture of an alkane and an alkene, an unsaturated carboxylic acid such as an α,β-unsaturated carboxylic acid can be obtained in good yield. For example, when propane and propene or isobutane and isobutene are used as the starting material mixture of alkane and alkene, acrylic acid or methacrylic acid will be obtained, respectively, in good yield. Preferably, in the mixture of alkane and alkene, the alkene is present in an amount of at least 0.5% by weight, more preferably at least 1.0% by weight to 95% by weight; most preferably, 3% by weight to 90% by weight.

As an alternative, an alkanol, such as isobutanol, which will dehydrate under the reaction conditions to form its corresponding alkene, i.e. isobutene, may also be used as a feed to the present process or in conjunction with the previously mentioned feed streams.

The purity of the starting material alkane is not particularly limited, and an alkane containing a lower alkane such as methane or ethane, air or carbon dioxide, as impurities, may be used without any particular problem. Further, the starting material alkane may be a mixture of various alkanes. Similarly, the purity of the starting material mixture of alkane and alkene is not particularly limited, and a mixture of alkane and alkene containing a lower alkene such as ethene, a lower alkane such as methane or ethane, air or carbon dioxide, as impurities, may be used without any particular problem. Further, the starting material mixture of alkane and alkene may be a mixture of various alkanes and alkenes.

There is no limitation on the source of the alkene. It may be purchased, per se, or in admixture with an alkane and/or other impurities. Alternatively, it can be obtained as a byproduct of alkane oxidation. Similarly, there is no limitation on the source of the alkane. It may be purchased, per se, or in admixture with an alkene and/or other impurities. Moreover, the alkane, regardless of source, and the alkene, regardless of source, may be blended as desired.

The detailed mechanism of the oxidation reaction of the present invention is not clearly understood, but the oxidation reaction is carried out by oxygen atoms present in the above mixed metal oxide or by molecular oxygen present in the feed gas. To incorporate molecular oxygen into the feed gas, such molecular oxygen may be pure oxygen gas. However, it is usually more economical to use an oxygen-containing gas such as air, since purity is not particularly required.

It is also possible to use only an alkane, or a mixture of alkane and alkene, substantially in the absence of molecular oxygen for the vapor phase catalytic reaction. In such a case, it is preferred to adopt a process wherein a part of the catalyst is appropriately withdrawn from the reaction zone from time to time, then sent to an oxidation regenerator, regenerated and then returned to the reaction zone for reuse. As the regeneration process of the catalyst, a process may, for example, be mentioned which comprises contacting an oxidative gas such as oxygen, air or nitrogen monoxide with the catalyst in the regenerator usually at a temperature of from 300° to 600°C.

A process for producing an unsaturated carboxylic acid may also be employed where propane is used as the starting material alkane, and air is used as the oxygen source. In such an instance, the reaction system may be preferably a fixed bed system. The proportion of air to be supplied to the reaction system is important for the selectivity for the resulting acrylic acid, and it is usually at most 25 moles, preferably from 0.2 to 18 moles per mole of propane, whereby high selectivity for acrylic acid can be obtained. This reaction can be conducted usually under atmospheric pressure, but may be conducted under a slightly elevated pressure or slightly reduced pressure. With respect to other alkanes such as isobutane, or to mixtures of alkanes and alkenes such as propane and propene, the composition of the feed gas may be selected in accordance with the conditions for propane.

Typical reaction conditions for the oxidation of propane or isobutane to acrylic acid or methacrylic acid may be utilized in the practice of the present invention. The process may be practiced in a single pass mode (only fresh feed is fed to the reactor) or in a recycle mode (at least a portion of the reactor effluent is returned to the reactor). General conditions for the process of the present invention are as follows: the reaction temperature can vary from 200°C to 700°C, but is usually in the range of from 200°C to 550°C, more preferably 250°C to 480°C, most preferably 300°C to 430°C; the gas space velocity, SV, in the vapor phase reaction is usually within a range of from 100 to 10,000 hr⁻¹, preferably 300 to 6,000 hr⁻¹, more preferably 300 to 3,000 hr⁻¹; the average contact time with the catalyst can be from 0.01 to 10 seconds or more, but is usually in the range of from 0.1 to 10 seconds, preferably from 0.2 to 6 seconds; the pressure in the reaction zone usually ranges from 0 to 75 psig, but is preferably no more than 50 psig. In a single pass mode process, it is preferred that the oxygen be supplied from an oxygen-containing gas. The term "oxygen-containing gas," as used herein, refers to any gas comprising from 0.01% up to 100% oxygen, including, for example, air. Thus, although the oxygen-containing gas may be pure oxygen gas, it is usually more economical to use an oxygen-containing gas such as air, since purity is not particularly required. The single pass mode process may also be practiced with oxygen addition. In the practice of the recycle mode process, oxygen gas by itself is the preferred source so as to avoid the build up of inert gases in the reaction zone.

Of course, in the oxidation reaction of the present invention, it is important that the hydrocarbon and oxygen concentrations in the feed gases be maintained at the appropriate levels to minimize or avoid entering a flammable regime within the reaction zone or especially at the outlet of the reactor zone. Generally, it is preferred that the outlet oxygen levels be low to both minimize after-burning and, particularly, in the recycle mode of operation, to minimize the amount of oxygen in the recycled gaseous effluent stream. In addition, operation of the reaction at a low temperature (below 450°C) is extremely attractive because after-burning becomes less of a problem, which enables the attainment of higher selectivity to the desired products. The catalyst of the present invention operates more efficiently at the lower temperature range set forth above, significantly reducing the formation of acetic acid and carbon oxides, and increasing selectivity to acrylic acid. As a diluting gas to adjust the space velocity and the oxygen partial pressure, an inert gas such as nitrogen, argon or helium may be employed.

When the oxidation reaction of propane, and especially the oxidation reaction of propane and propene, is conducted by the process of the present invention, carbon monoxide, carbon dioxide, acetic acid, etc. may be produced as by-products, in addition to acrylic acid. Further, in the process of the present invention, an unsaturated aldehyde may sometimes be formed depending upon the reaction conditions. For example, when propane is present in the starting material mixture, acrolein may be formed; and when isobutane is present in the starting material mixture, methacrolein may be formed. In such a case, such an unsaturated aldehyde can be converted to the desired unsaturated carboxylic acid by subjecting it again to the vapor phase catalytic oxidation with the promoted mixed metal oxide-containing catalyst of the present invention or by subjecting it to a vapor phase catalytic oxidation reaction with a conventional oxidation reaction catalyst for an unsaturated aldehyde.

In yet another aspect, the process of the present invention comprises subjecting an alkane, or a mixture of an alkane and an alkene, to a vapor phase catalytic oxidation reaction with ammonia in the presence of a supported catalyst containing the above mixed metal oxide, to produce an unsaturated nitrile.

In the production of such an unsaturated nitrile, as the starting material alkane, it is preferred to employ a C₃₋₈ alkane such as propane, butane, isobutane, pentane, hexane and heptane. However, in view of the industrial application of nitriles to be produced, it is preferred to employ a lower alkane having 3 or 4 carbon atoms, particularly propane and isobutane.

Similarly, as the starting material mixture of alkane and alkene, it is preferred to employ a mixture of C₃₋₈ alkane and C₃₋₈ alkene such as propane and propene, butane and butene, isobutane and isobutene, pentane and pentene, hexane and hexene, and heptane and heptene. However, in view of the industrial application of nitriles to be produced, it is more preferred to employ a mixture of a lower alkane having 3 or 4 carbon atoms and a lower alkene having 3 or 4 carbon atoms, particularly propane and propene or isobutane and isobutene. Preferably, in the mixture of alkane and alkene, the alkene is present in an amount of at least 0.5% by weight, more preferably at least 1.0% by weight to 95% by weight, most preferably 3% by weight to 90% by weight.

The purity of the starting material alkane is not particularly limited, and an alkane containing a lower alkane such as methane or ethane, air or carbon dioxide, as impurities, may be used without any particular problem. Further, the starting material alkane may be a mixture of various alkanes. Similarly, the purity of the starting material mixture of alkane and alkene is not particularly limited, and a mixture of alkane and alkene containing a lower alkene such as ethene, a lower alkane such as methane or ethane, air or carbon dioxide, as impurities, may be used without any particular problem. Further, the starting material mixture of alkane and alkene may be a mixture of various alkanes and alkenes.

There is no limitation on the source of the alkene. It may be purchased, per se, or in admixture with an alkane and/or other impurities. Alternatively, it can be obtained as a byproduct of alkane oxidation. Similarly, there is no limitation on the source of the alkane. It may be purchased, per se, or in admixture with an alkene and/or other impurities. Moreover, the alkane, regardless of source, and the alkene, regardless of source, may be blended as desired.

The detailed mechanism of the ammoxidation reaction of this aspect of the present invention is not clearly understood. However, the oxidation reaction is conducted by the oxygen atoms present in the above mixed metal oxide or by the molecular oxygen in the feed gas. When molecular oxygen is incorporated in the feed gas, the oxygen may be pure oxygen gas. However, since high purity is not required, it is usually economical to use an oxygen-containing gas such as air.

As the feed gas, it is possible to use a gas mixture comprising an alkane, or a mixture of an alkane and an alkene, ammonia and an oxygen-containing gas, however, a gas mixture comprising an alkane or a mixture of an alkane and an alkene and ammonia, and an oxygen-containing gas may be supplied alternately.

When the gas phase catalytic reaction is conducted using an alkane, or a mixture of an alkane and an alkene, and ammonia substantially free from molecular oxygen, as the feed gas, it is advisable to employ a process wherein a part of the catalyst is periodically withdrawn and sent to an oxidation regenerator for regeneration, and the regenerated catalyst is returned to the reaction zone. As a process for regenerating the catalyst, a process may be mentioned wherein an oxidizing gas such as oxygen, air or nitrogen monoxide is permitted to flow through the catalyst in the regenerator usually at a temperature of from 300°C to 600°C.

A process may also be employed where propane is used as the starting material alkane, and air is used as the oxygen source. The proportion of air to be supplied for the reaction is important with respect to the selectivity for the resulting acrylonitrile. Namely, high selectivity for acrylonitrile is obtained when air is supplied within a range of at most 25 moles, particularly 1 to 15 moles, per mole of the propane. The proportion of ammonia to be supplied for the reaction is preferably within a range of from 0.2 to 5 moles, particularly from 0.5 to 3 moles, per mole of propane. This reaction may usually be conducted under atmospheric pressure, but may be conducted under a slightly increased pressure or a slightly reduced pressure. With respect to other alkanes such as isobutane, or to mixtures of alkanes and alkenes such as propane and propene, the composition of the feed gas may be selected in accordance with the conditions for propane.

The process of the third aspect of the present invention may be conducted at a temperature of, for example, from 250°C to 480°C. More preferably, the temperature is from 300°C to 400°C. The gas space velocity, SV, in the gas phase reaction is usually within the range of from 100 to 10,000 hr⁻¹, preferably from 300 to 6,000 hr⁻¹, more preferably from 300 to 2,000 hr⁻¹. As a diluent gas, for adjusting the space velocity and the oxygen partial pressure, an inert gas such as nitrogen, argon or helium can be employed. When ammoxidation of propane is conducted by the process of the present invention, in addition to acrylonitrile, carbon monoxide, carbon dioxide, acetonitrile, hydrocyanic acid and acrolein may form as by-products.

### Comparative Examples

First, comparative exemplary catalysts were made using a rotovap drying step. A catalyst of nominal composition Mo_{1.0}V_{0.3}Te_{0.23}Nb_{0.22}Pd_{0.01}Oₓ was prepared in the following manner: 100 mL of an aqueous solution containing ammonium heptamolybdate tetrahydrate (1.0M Mo), ammonium metavanadate (0.3M V) and telluric acid (0.23M Te) formed by dissolving the corresponding salts in water at 70°C, was added to a 1000 mL rotovap flask. Then 100 mL of an aqueous solution of ammonium niobium oxalate (0.22M Nb), palladium nitrate hydrate (0.01M Pd), oxalic acid (0.155M) and nitric acid (0.24M) were added thereto.

After removing the water via a rotary evaporator with a warm water bath at 50°C and 28 mm Hg, the solid materials were further dried in a vacuum oven at 25°C overnight and then calcined. Calcination was effected by placing the solid materials in an air atmosphere and then heating them to 275°C at 10°C/min and holding them under the air atmosphere at 275°C for one hour; the atmosphere was then changed to argon and the material was heated from 275°C to 600°C at 2°C/min and the material was held under the argon atmosphere at 600°C for two hours. The calcined materials were ground with a high-speed micro-mill (MS 100 from Retsch), then refluxed with 1.5% oxalic acid in methanol for 5 hours. The solids were collected by gravity filtration and dried in vacuum oven overnight. The catalyst, thus obtained, was pressed and sieved to 14 - 20 mesh granules for reactor evaluation.

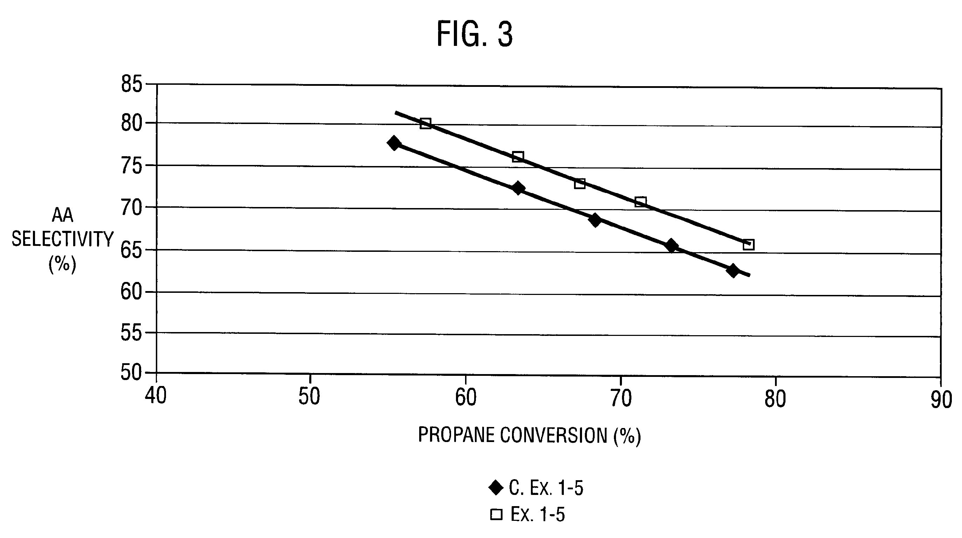

Four grams (4g) of these granules were packed into a stainless steel tube reactor (inside diameter: 1.1 cm) for the gas phase oxidation of propane. The reactor was heated with an electric furnace and fed with a mixture of propane, air and steam having a feed composition of 4% propane, 9.6% oxygen, 63.4% nitrogen and 23% steam. The effluent of the reactor was analyzed by gas chromatography to determine the propane conversion and the yield of acrylic acid. The results along with residence time and reactor temperature are shown in Table 1 and Figure 3.

**TABLE 1**

| Examples | Residence Time (sec) | Temperature (°C) | Propane Conversion (%) | Acrylic Acid Selectivity (%) | Acrylic Acid Yield (%) |
|---|---|---|---|---|---|
| C. Ex. 1 | 3 | 358 | 55 | 78 | 43 |
| C. Ex. 2 | 3 | 366 | 63 | 73 | 46 |
| C. Ex. 3 | 3 | 372 | 68 | 69 | 47 |
| C. Ex. 4 | 3 | 378 | 73 | 66 | 48 |
| C. Ex 5 | 3 | 384 | 77 | 63 | 49 |

### Examples

Second, exemplary catalysts were made using a SFE drying step. A catalyst of nominal composition Mo_{1.0}V_{0.3}Te_{0.23}Nb_{0.22}Pd_{0.01}Oₓ was prepared in the following manner: 70 mL of an aqueous solution containing ammonium heptamolybdate tetrahydrate (1.0M Mo), ammonium metavanadate (0.3M V) and telluric acid (0.23M Te) formed by dissolving the corresponding salts in water at 70°C, was added to a 500 mL beaker. Then 70 mL of an aqueous solution of ammonium niobium oxalate (0.17M Nb), palladium nitrate hydrate (0.01M Pd), oxalic acid (0.155M) and nitric acid (0.24M) were added thereto.

An orange colored gel was formed. The gel was first extracted with 100 mL of ethanol three times to form an alcogel, then the alcogel was transferred to a high pressure reactor and extracted with supercritical carbon dioxide at 60°C and 200 bars pressure for 3 hrs. The dried solid material from the high pressure reactor was calcined by placing them in an air atmosphere and then heating them to 275°C at 10°C/min and holding them under the air atmosphere at 275°C for one hour; the atmosphere was then changed to argon and the material was heated from 275°C to 600°C at 2°C/min and the material was held under the argon atmosphere at 600°C for two hours. The calcined materials were ground with a high-speed micro-mill (MS 100 from Retsch), then refluxed with 1.5% oxalic acid in methanol for 5 hours. The solids were collected by gravity filtration and dried in vacuum oven overnight. The catalyst, thus obtained, was pressed and sieved to 14 - 20 mesh granules for reactor evaluation.

Four grams (4g) of these granules were packed into a stainless steel tube reactor (inside diameter: 1.1 cm) for the gas phase oxidation of propane. The reactor was heated with an electric furnace and fed with a mixture of propane, air and steam having a feed composition of 4% propane, 9.6% oxygen, 63.4% nitrogen and 23% steam. The effluent of the reactor was analyzed by gas chromatography to determine the propane conversion and the yield of acrylic acid. The results along with residence time and reactor temperature are shown in Table 2 and Figure 3.

**TABLE 2**

| Examples | Residence Time (sec) | Temperature (°C) | Propane Conversion (%) | Acrylic Acid Selectivity (%) | Acrylic Acid Yield (%) |
|---|---|---|---|---|---|
| Ex. 1 | 3 | 341 | 57 | 80 | 46 |
| Ex. 2 | 3 | 347 | 63 | 76 | 48 |
| Ex. 3 | 3 | 351 | 67 | 73 | 49 |
| Ex. 4 | 3 | 357 | 71 | 71 | 51 |
| Ex. 5 | 3 | 362 | 78 | 66 | 52 |

### Additional Examples

Atomization was carried out in a system manufactured by Thar Technologies (Pittsburgh, PA), as generally shown in Fig. 2. Two aqueous precursor solutions (Solutions A and B) were prepared. The composition of Solutions A and B are as follows: Solution A: M0=1M, V=0.3M, and Te=0.23M; and Solution B: Nb=0.17M, oxalic acid=0.155M, Pd=0.01M and HNO₃=0.24M. CO₂ (purity, 99.5 %) was obtained from Greco Gas and Welding (Tarentum, USA) for use as the supercritical stream and organics solvents, ethanol (purity, 90%) and methanol (purity, 99%) from LabChem (Pittsburgh, PA).

The carbon dioxide and the pump heads were chilled (5°C) to avoid cavitation and compressibility problems. The liquid CO₂ was compressed by means of the high-pressure pump 16 to the operating pressure at constant flow rate. The high-pressure CO₂ flowed through a pre-heater 18 to ensure that it reached the extraction temperature before contact with the vessel 24 (1000 ml). Once the vessel 24 reached the operating conditions, the solutions (A and B) were pumped into the system. The solutions were mixed before contact with the supercritical stream (CO₂ + ethanol). Therefore, any gelation process would take place before atomization and drying. The precursor was mixed with the supercritical stream that acts as "spray enhancer" by mechanical effects. The high velocity of the supercritical stream allows the break up of the precursor into very small droplets. Moreover, the supercritical stream can extract the solvent from the solution as it meets and disperses the solution. The solution mixture and the water left the vessel from the bottom and passed through a pressure reduction valve 24 and into a separation vessel 26, where the ethanol and water were collected.

The experiments were carried out at a constant pressure (200 bar) and CO₂ flow rate (75 g/min) using two different temperatures (40°C and 60°C) and two different amounts of ethanol as a co-solvent (4 and 6 ml/min). The solutions (A and B) flow rates were equal and constant at 0.5 ml/min. After extraction, the solutions and co-solvent pumps were stopped and CO₂ was used independently to remove any residual ethanol in the material.

Different parameters were tested to determine which influenced the quality of the catalysts and the operation of the system, e.g. gelation distance, coaxial space for gel formation, mixing area with supercritical stream before spraying and spray nozzle type. Modifying these parameters helped reduce clogging of the nozzle.

The following insights may be concluded from these additional examples of gel atomization: higher temperatures lead to drier material; increasing gelation distances leads to clogging of the lines; reducing the nozzle size decreases the particle size.

It will be further appreciated that functions or structures of a plurality of components or steps may be combined into a single component or step, or the functions or structures of one step or component may be split among plural steps or components. The present invention contemplates all of these combinations. Unless stated otherwise, dimensions and geometries of the various structures depicted herein are not intended to be restrictive of the invention, and other dimensions or geometries are possible. Plural structural components or steps can be provided by a single integrated structure or step. Alternatively, a single integrated structure or step might be divided into separate plural components or steps. In addition, while a feature of the present invention may have been described in the context of only one of the illustrated embodiments, such feature may be combined with one or more other features of other embodiments, for any given application. It will also be appreciated from the above that the fabrication of the unique structures herein and the operation thereof also constitute processes in accordance with the present invention.

The explanations and illustrations presented herein are intended to acquaint others skilled in the art with the invention, its principles, and its practical application. Those skilled in the art may adapt and apply the invention in its numerous forms, as may be best suited to the requirements of a particular use. Accordingly, the specific embodiments of the present invention as set forth are not intended as being exhaustive or limiting of the invention. The scope of the invention should, therefore, be determined not with reference to the above description, but should instead be determined with reference to the appended claims, along with the full scope of equivalents to which such claims are entitled. The disclosures of all articles and references, including patent applications and publications, are incorporated by reference for all purposes.

## Claims

1. A process for preparing an improved catalyst comprising:
admixing compounds containing components of the catalyst and at least one solvent to form a precursor;
extracting the precursor to form a processed precursor; and
calcining the processed precursor to form a catalyst,
wherein the extracting step comprises drying the precursor with a supercritical stream, atomizing the precursor, and combinations thereof.

2. The process of claim 1, wherein the extraction step comprises drying the precursor by introducing the precursor, which has been previously washed with an alcohol, into a vessel and introducing the supercritical stream at a pressure and temperature above the critical point of the supercritical stream, and wherein the catalyst formed is a mixed metal oxide catalyst.

3. The process of claim 1, wherein the extracting step comprises drying and atomizing the precursor by at least one method selected from the group consisting of introducing the supercritical stream into the vessel at a pressure and a temperature above critical point of the supercritical stream, introducing the precursor into the vessel through a nozzle, and introducing the precursor and the supercritical stream into the vessel through a nozzle.

4. The process of claim 3, wherein the supercritical stream is selected from the group consisting of carbon dioxide, water, ammonia, nitrogen, nitrous oxide, methane, ethane, ethylene, propane, butane, n-pentane, benzene, methanol, ethanol, isopropanol, isobutanol, monofluoromethane, trifluoromethane, chlorotrifluoromethane, monofluoromethane, hexafluoroethane, 1,1-difluoroethylene, 1,2-difluoroethylene,toluene, pyridine, cyclohexane, m-cresol, decalin, cyclohexanol, o-xylene, tetralin, aniline, acetylene, chlorotrifluorosilane, xenon, sulfur hexafluoride, propane, and combinations thereof.

5. The process of claim 3, wherein the precursor is formed as a slurry, a solution, a colloidal suspension, a gel, or a combination thereof.

6. A catalyst for the vapor phase catalytic oxidation of alkanes or a mixture of alkanes and alkenes to their corresponding unsaturated carboxylic acids or unsaturated nitriles prepared by the process of claim 3.

7. The catalyst of claim 6, comprising a mixed metal oxide having the empirical formula: AₐD_{b}E_{c}X_{d}Oₑ, wherein A is at least one element selected from the group consisting of Mo and W, D is at least one element selected from the group consisting of V and Ce, E is at least one element selected from the group consisting of Te, Sb and Se, and X is at least one element selected from the group consisting of Nb, Ta, W, Ti, Al, Zr, Cr, Mn, Fe, Ru, Co, Rh, Ni, Pt, Sb, Bi, B, In, Ce, As, Ge, Sn, Li, Na, K, Rb, Cs, Fr, Be, Mg, Ca, Sr, Ba, Ra, Hf, Ag, Pb, P, Pm, Eu, Gd, Dy, Ho, Er, Tm, Yb, Lu and mixtures thereof, and wherein a, b, c, d, and e represent the atomic ratios of A, D, E, X and O, respectively, and the stoichiometry of the mixed metal oxide is selected such that when a=0.01-1.0, b=0.003-1.0, c=0.01-1.0, d=0-1.0, and e is a number such that the total valency of the metal elements is satisfied.

8. The catalyst of claim 7, wherein the mixed metal oxide has an empirical formula: MoₐV_{b}E_{c}X_{d}Oₑ, wherein E is at least one element selected from the group consisting of Te and Sb, X is at least one element selected from the group consisting of Nb, Ta, Ti, Al, Zr, Cr, Mn, Fe, Ru, Co, Rh, Ni, Pd, Pt, Ag, B, Bi, In, Ce and W and mixtures thereof, and wherein a, b, c, d, and e represent the atomic ratios of A, D, E, X and O, respectively, and when a=1, b=0.01-1.0, c=0.01-1.0, d=0.01-1.0, and e is a number such that the total valency of the metal elements is satisfied.

9. A process for preparing an unsaturated carboxylic acid comprising subjecting an alkane, or a mixture of an alkane and an alkene, to vapor phase catalytic oxidation in the presence of a catalyst prepared by the process of claim 3.

10. A process for preparing an unsaturated nitrile comprising subjecting an alkane, or a mixture of an alkane and an alkene, to vapor phase catalytic oxidation in the presence of ammonia and a catalyst prepared by the process of claim 3.

11. A system for carrying out the process of claim 3, comprising:
a source of catalyst precursor in solution;
a source of a supercritical stream;
a vessel; and
an atomizer connected to the catalyst precursor source and adapted to introduce the catalyst precursor into the vessel.

12. The system of claim 11, wherein the system further comprises a point of introduction of the catalyst precursor into the vessel, and wherein the source of catalyst precursor and the source of a supercritical stream are connected to each other upstream from the point of introduction.

13. The system of claim 11, wherein the source of a supercritical stream is connected to the vessel.
